# EUROPÄISCHE PATENTSCHRIFT

(11) **EP 0 287 823 B1**
(45) Veröffentlichungstag und Bekanntmachung des Hinweises auf die Patenterteilung: **21.07.1993**
(21) Anmeldenummer: 88104527.2
(22) Anmeldetag: 22.03.1988
(51) Int. Cl.: A61B 17/58, B25B 15/04

(54) **Chirurgisches Handinstrument**
Surgical handtool
Instrument chirurgical

(30) Priorität: 25.03.1987 DE 3709824
(43) Veröffentlichungstag der Anmeldung: 26.10.1988
(73) Patentinhaber: Heinl, Thomas, Dr., D-32427 Minden (DE)
(72) Erfinder: Heinl, Thomas, Dr., D-32427 Minden (DE)
(74) Vertreter: Pöhner, Wilfried Anton, Dr.

(56) Entgegenhaltungen:
- DE-C- 39 923
- GB-A- 196 759
- US-A- 2 486 043
- US-A- 4 517 865

## Beschreibung

Die Erfindung bezieht sich auf ein chirurgisches Handinstrument insbesondere zur Osteosynthese gemäß dem Oberbegriff des Anspruches 1.

Aus der Chirurgie ist bekannt, die durch Unfälle oder operative Eingriffe erzeugten Knochenfragmente in der gewollten, präzise zueinander ausgerichteten Position stabil zu fixieren, um nach der Heilung und der Zusammenwachsung das gewünschte Resultat zu erhalten. Dazu werden in fast allen chirurgischen Disziplinen, z.B. Chirurgie, HNO, Orthopädie, Kiefer-, Neuro-, Hand- und Unfallchirurgie Platten eingesetzt, welche mit den Knochenfragmenten verschraubt und nach erfolgter Heilung durch einen erneuten Eingriff wieder entfernt werden. Hierzu sowie zu anderen Zwecken finden die aus dem Maschinenbau allgemein bekannten Schraubenzieher, Bohrwerkzeuge und Gewindeschneide Verwendung. Zur Reduzierung der Belastung des Patienten ist eines der vordringlichsten Ziele, die Operationszeit möglichst kurz zu halten. Ein Schraubendreher gattungsgemäßer Art ist aus der DE-C-39923 bekannt, bei der im Griff koaxial eine Welle drehbar angebracht, an deren äußerer Stirnseite das Werkzeug befestigt ist. Auf dieser Welle befinden sich innerhalb des Griffes zwei Zahnräder im Abstand sowie eine von außen betätigbare Sperrklinke. In der einen Endlage greift die Sperrklinke in den Zahnkranz des einen Zahnrades ein und gibt das andere Zahnrad frei, wohingegen umgekehrt, d.h. in der anderen Endlage in den Zahnkranz des anderen Zahnrades eingegriffen wird. Entscheidend ist, daß die Sperrung der beiden Zahnräder in entgegengesetzter Drehrichtung vorgenommen wird. Bei Eingriff der Sperrklinke in den Zahnkranz wird damit das Zahnrad an der Rotation in der einen Richtung gehindert; bei Bewegung in entgegengesetzten Richtung wird die Sperrklinke nach außen weggedrückt und deshalb eine Bewegung zugelassen. Als Resultat erhält man eine Sperrung der Rotation in der einen und eine Freigabe in der anderen entgegengesetzten Richtung. Der jeweilige Drehsinn wird bestimmt durch die Stellung der Sperrklinke, durch deren Betätigen eine Umkehrung der Bewegung möglich wird.
Dadurch läßt sich beispielsweise beim Eindrehen von Schrauben durch die entsprechende Position der Sperrklinke erreichen, daß bei der einen üblicherweise Rechtsdrehung zum Einbringen der Schrauben die Welle durch die Sperrklinke arretiert ist, so daß die Drehung des Griffes auf die Klinge bzw. Schraube übertragen wird. Bei einer Drehung in die entgegengesetzten Richtung bleibt Schraubenzieherklinge und Schraube raumfest und nur der Griff bewegt sich. Umgekehrt kann die gleiche Schraube dadurch gelöst werden, daß man nach Umstellen der Sperrklinke bei der normalerweise dann erforderlichen Linksdrehung eine Sperrung der Welle erhält, so daß die Drehbewegung unmittelbar auf die Schraube übertragen und diese herausgedreht werden kann. Bei Bewegen in der entgegengesetzten Richtung entsteht ein Leerlauf relativ zwischen Griff und Schraubenzieherklinge und die Klinge bleibt raumfest.

Der Nachteil dieser Anordnung ist darin zu sehen, daß die Sperrung des Zahnrades unmittelbar über den Schieber erfolgt, der so zu dimensionieren ist, daß er zur Aufnahme der beim Betätigen anfallenden Kräfte in der Lage ist. Zudem ist aufgrund der unmittelbaren Einwirkung der Kräfte nur ein schwergängiges Betätigen des Schiebers möglich.

Hiervon ausgehend hat sich die Erfindung die Verbesserung der in der Chirurgie verwendeten Handinstrumente dahingehend zur Aufgabe gemacht, daß sie ein schnelleres Arbeiten ermöglichen.

Gelöst wird diese Aufgabe erfindungsgemäß dadurch, daß die Sperrklinke aus jeweils um eine sehnenartig im Griff verlaufende Achse verschwenkbares und jeweils in ein Zahnrad eingreifendes Plättchen sowie einem von außen im Griff bewegbaren Schieber besteht, der in der Endposition jeweils ein Plättchen aus dem Zahnkranz herausschwenkt.

Die Sperrklinke besteht aus Plättchen, die jeweils um eine sehnenartig im Griff verlaufende Achse verschwenkbar sind und in ein Zahnrad eingreifen. Die Anzahl der Plättchen wird bestimmt durch die Gesamtzahl der Zahnräder bzw. Zahnradwalzen. Zur Verstellung ist im Griff bewegbar ein Schieber angeordnet, dessen Form bedingt, daß in der jeweiligen Endposition ein Plättchen aus dem Zahnkranz herausgeschwenkt wird. Das andere Plättchen hingegen steht in Eingriff mit dem Zahnkranz und bewirkt bei Bewegung im einen Drehsinn eine Sperrung und wird bei Drehung in entgegengesetzte Richtung abgehoben, so daß Zahnrad und damit auch Welle ungehindert bewegt werden können.

Vorliegende Erfindung befaßt sich mit der Ausbildung des Griffes. Deshalb ist grundsätzlich unerheblich, welches konkrete Werkzeug daran befestigt ist bzw. werden kann. Die soeben anhand eines Schraubenziehers beispielhaft erläuterte Funktion läßt sich in gleicher Weise an Werkzeugen wie Gewindeschneidern, Bohrer durch Befestigung an der Welle des Griffes realisieren. Der Zahnkranz beider Zahnräder besitzt im speziellen ein Sägezahnprofil, d.h. ein Profil, bei dem die eine Flanke im wesentlichen in radialer Richtung verläuft und die andere in einem spitzen Winkel hierzu, wobei die Flanken gleichen Anstiegs auf entgegengesetzten Seiten liegen.

Der entscheidende Vorteil der Erfindung liegt darin, daß hierdurch ein Ein- und Herausdrehen sehr einfach und rasch deshalb durchgeführt werden kann, weil der Benutzer den Griff nicht mehr loslassen, umgreifen und anschließend die Schraubbewegung fortsetzen muß. Es ist nicht mehr erforderlich, daß das Werkzeug während des Umgreifens mit der anderen Hand festgehalten werden muß, so daß in vorteilhafter Weise ein vollständiges Eindrehen unter Zuhilfenahme nur einer einzigen Hand möglich wird. Das Ein- und auch Herausdrehen der Schrauben ergibt sich durch eine oszillatorische Bewegung des Griffes, wobei bei Betätigung im einen Drehsinn die Klinge bewegt und im entgegengesetzten Sinne raumfest läßt. Eine Umkehrung der jeweiligen Sperr- bzw. Freigaberichtung erhält man durch Betätigen der Sperrklinke. Aufgrund der Tatsache, daß die Kräfte durch die Plättchen aufgenommen werden, also die Zahnräder über die Plättchen selbst eine Abstützung erfahren, ist der Schieber wenig belastet und deshalb rasch und leichtgängig betätigbar. Des weiteren kann die Herstellung aufgrund der Einfachheit des Aufbaues der einzelnen Elemente preisgünstig erfolgen.

In einer konkreten Ausbildung besteht der Schieber aus zwei Feder- oder Magnetelementen, die sich in jeder Endposition gegen wenigstens ein Plättchen pressen und dieses hierdurch aus dem Zahnkranz herausschwenken. Um das vorgeschlagene Handinstrument in der gleichen Weise wie einen herkömmlicher Schraubenzieher, d.h. also mit zum Griff und unabhängig von der Drehrichtung fester Klinge verwendbar zu machen, ist es zweckmäßig, den Schieber so auszugestalten, daß dann alle Plättchen in den Zahnkranz eingreifen. Hierdurch wird die Welle und folglich das mit diesem verbundene Werkzeug im Griff arretiert.

In einer zweckmäßigen Weiterbildung ist vorgeschlagen, die Schrittweite der Zahnkränze beider Zahnräder unterschiedlich zu wählen. Diese Weiterbildung der Erfindung sieht es als ideal an, beim Eindrehen von Schrauben die Schrittweite relativ eng zu wählen, so daß ein feinfühliges Ein- und Festschrauben durch den geringen Abstand der einzelnen Zähne des Zahnkranzes voneinander ermöglicht wird. Allerdings bedingt die geringe Schrittweite die Ausbildung entsprechend schmaler und schlanker Zähne, deren Festigkeit und maximale Belastbarkeit dann vergleichsweise gering ist. Für das Hineindrehen der Schrauben sind relativ geringe Kräfte aufzuwenden, jedoch ist ein feinfühliges und dosiertes Einschrauben von erheblichem Vorteil. Im Gegensatz dazu sollen beim Herausdrehen der Schrauben große Momente aufgebracht werden können. Ein feinfühliges Arbeiten und damit kleine Breiten der einzelnen Zähne sind nicht erforderlich. Aus diesem Grund wird man das eine, für das Hineindrehen benutzte Zahnrad mit einer vergleichsweise kleinen, hingegen das andere zum Herausdrehen genutzte, mit einer relativ großen Schrittweite versehen.

In besonders vorteilhafter Weise werden enge Schrittweiten konkret dadurch verwirklicht, daß an Stelle eines Zahnrades zwei koaxiale Zahnradwalzen mit gegeneinander azimutal versetzten Zahnkränzen eingebaut werden, denen jeweils eine Sperrklinke für jede Drehrichtung zugeordnet ist. Hierdurch lassen sich unter Beibehaltung der Form der Zahnkränze der einzelnen Zahnradwalzen eine effektive Verringerung und insbesondere Halbierung der Schrittweiten erreichen. Aufgrund der Beibehaltung der Form der Zahnkränze ist die mögliche Kraft zum Abstützen und die maximale Belastbarkeit ungeändert, obwohl sich die Schrittweiten verringern. Dieses Paar von Zahnradwalzen kann an die Stelle eines oder beider Zahnräder treten.

Bei in azimutaler Richtung federnder Lagerung wenigstens einer der Zahnradwalzen ergibt sich bei Anliegen an der Sperrklinke eine durch die Reaktionskraft entgegen der Bewegung gerichtete Verschiebung der Zahnradwalze, so daß nach hinreichend weiter Bewegung in azimutaler Richtung d.h. Verdrehung der Zahnradwalze relativ zur Welle, auch die andere Zahnradwalze ihrerseits an der Sperrklinke zur Anlage kommt, so daß trotz azimutal versetzter Anordnung dann beide Zahnkränze anliegen, die aufzubringenden Kräfte verteilen und so einer Überlastung entgegengewirkt wird. Mit Beendigung der Anlage des Zahngrenze an der Sperrklinke kehrt die Zahnradwalze aufgrund federnder Lagerung wieder in ihre Ausgangsposition zurück. Eine derartige Anordnung vereinigt zum einen die Möglichkeit der feinen Abstufung und zum anderen bei Belastung in Sperrichtung eine optimale Abstützung und deshalb maximale Belastbarkeit. Hierbei reicht es in aller Regel aus, die azimutale Bewegung der Zahnradwalze zu begrenzen und den Zahnabstand maximal zu wählen.

Zur Erleichterung des Umschaltens der Sperrklinke ist die Betätigung im Vorderteil des Griffes angeordnet. Ohne Lösen und Freigeben des Griffes ist dann ein Umschalten und Vertauschen des Drehsinnes vornehmbar.

Während der Operationen ist besonderes Augenmerk darauf zu richten, daß die Schraube nicht überlastet und Brüche provoziert werden. Ziel ist es, das Anzugsmoment eine bestimmte Grenze, die abhängig ist von der Knochenstruktur unter Länge und Durchmesser der Schraube, nicht zu überschreiten. Aus diesem Grunde ist in einer besonders bevorzugten Ausführungsform die Welle mit einem Drehmomentbegrenzer verbunden. In an sich bekannter Weise besteht seine Aufgabe darin, mit Überschreiten eines bestimmten Drehmoments die Verbindung zwischen Griff und Welle zu trennen und das auf den Griff ausgeübte Drehmoment nicht auf die Klinge weiterzugeben.

Aus der großen Zahl der grundsätzlich denkbaren Realisierungsmöglichkeiten wird durch die Erfindung ein Drehmomentbegrenzer vorgeschlagen, der aus zwei gegeneinander gepreßten Kupplungsscheiben besteht, von der eine mit der Welle und die andere mit dem Griff verbunden ist. Durch zum Teil schon geringfügige Verschiebungen der Kupplungsscheiben in axialer Richtung gegeneinander wird die Verbindung gelöst und es erfolgt eine Trennung der Welle vom Griff.

Insbesondere können die Kupplungsscheiben mit aufeinander zu weisendem Zahnprofil versehen sein, das im Spezialfall des Sägezahnprofiles bei Verdrehen nur in eine Richtung anspricht, wobei die Kupplungsscheiben durch Federn gegeneinander gepreßt werden. Die Richtung des Sägezahnprofiles wird man so wählen, daß der Drehmomentenbegrenzer nur beim Einschrauben anspricht, beim Herausdrehen, also in Gegenrichtung jedoch außer Funktion gesetzt ist. Bei einer anderen Realisierung der Kupplung greifen federbelastete Kugeln in Nuten oder Zahnkränzen ein.

Eine Änderung des auslösenden Momentes läßt sich realisieren, wenn die Vorspannung der die Kupplungsscheiben aufeinanderpressenden Feder verstellt wird, beispielsweise dadurch, daß das Gegenlager der Feder relativ zur Kupplung verschoben wird, so daß die Feder einer Änderung ihrer Länge und im Gefolge davon ihrer Spannung erfährt.

Schließlich ist noch als vorteilhaft erkannt, auf der Welle einen Schnellverschluß für die Anbringung der Werkzeuge also Schraubenzieherklinge, Gewindeschneider, Bohrer usw. vorzusehen, die ein rasches Austauschen der Werkzeuge ermöglicht. Die Realisierung kann in ansich bekannter Weise über einen Klemm-, Magent- oder über in eine Ringnut eingreifende und unter Vorspannung stehender Kugeln verwirklicht werden.

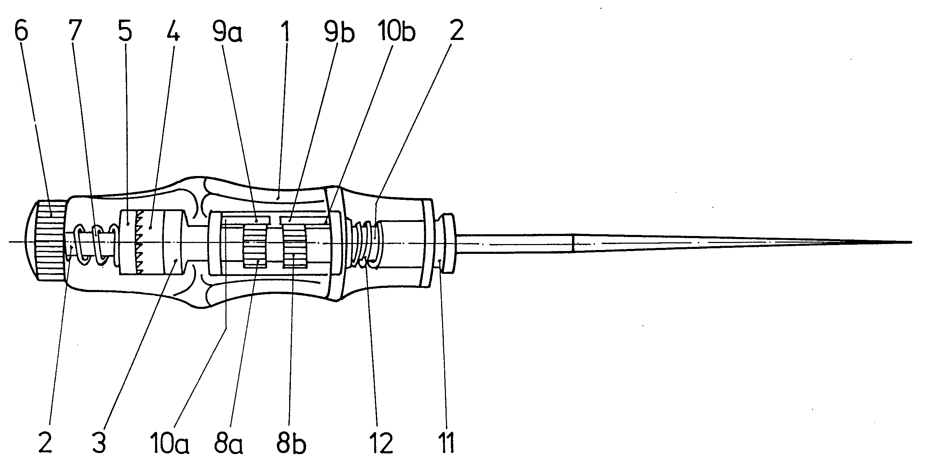

Weitere Einzelheiten, Merkmale und Vorteile der Erfindung lassen sich dem nachfolgenden Beschreibungsteil entnehmen, in dem anhand der Zeichnung ein Ausführungsbeispiel der Erfindung näher erläutert wird. Sie zeigt einen erfindungsgemäßen Schraubenzieher bei teilweise geöffnetem Griff in Seitenansicht.

Zu sehen ist in Seitenansicht der Schraubenzieher mit geöffnetem Griff 1, so daß der das eigentliche Wesen der Erfindung ausmachende innere Mechanismus zu sehen ist. Man erkennt eine drehbar innerhalb des Griffes und diesen vollständig durchgreifende Welle 2. Etwa im linken Drittel ist die Welle 2 insofern unterbrochen als angrenzend an ein Kugellager 3 eine vordere 4 und hintere Kupplungsscheibe 5 aufeinander zu weisend, hierbei die hintere Kupplungsscheibe 5 in Richtung der Welle 2 verschlieblich befestigt sind. Am linken Ende des Griffes 1 befindet sich eine Verstellschraube 6, deren Aufgabe darin besteht, die sich zwischen dieser und der hinteren Kupplungsscheibe 5 befindliche Feder 7 in ihrer Vorspannung zu verändern und zu beeinflussen. Die Spannung der Feder 7 bestimmt den Anpreßdruck der hinteren 5 gegen die vordere Kupplungsscheibe 4. Überschreitet das vermittels des Griffes 1 ausgeübte Drehmoment einen bestimmten Wert, endet der Reibungsschluß zwischen den beiden Kupplungsscheiben 4, 5 und es erfolgt eine Trennung, so daß zwar der Griff 1 bewegt, die Welle 2 jedoch nicht mitgeführt wird. Dieses maximal mögliche ausübbare Drehmoment wird bestimmt durch die Vorspannung der Feder 7 und ist in der Größe durch die Verstellschraube 6 einstellbar.

Etwa im mittleren Drittel des Griffes 1 befindet sich der für vorliegende Erfindung entscheidende Teil. Er besteht aus zwei im Abstand zueinander befindlichen und auf der Welle 2 befestigte Zahnräder 8a, 8b. Beide unterscheiden sich durch unterschiedlich geformten Zahnkranz, d.h. beide weisen ein Sägezahnprofil auf, das jedoch gegeneinander orientiert ist, daß also die Flanken gleichen Anstiegs bei beiden Zahnrädern 8a, 8b auf entgegengesetzten Seiten liegen.

Jedem Zahnrad 8a, 8b ist jeweils in Plättchen 9a, 9b zugeordnet, das in Achsenrichtung der Welle 2 verlaufend in den zugehörigen Zahnkranz eingreift. Beide Plättchen sind um eine segmentartig zur Welle 2 bzw. Griff 1 verlaufende Achse 10 a,b verschwenkbar, die es erlaubt, das jeweilige Plättchen 9 aus dem Zahnkranz des Zahnrades 8 herauszuheben und zur Rotation frei zu geben. Gemäß der Erfindung befindet sich, was hier jedoch nicht zu erkennen ist, jeweils nur eines der beiden Plättchen 9 im Eingriff mit zugehörigem Zahnrad 8. Hierdurch wird die Rotation der Welle 2 in Abhängigkeit vom Sägezahnprofil des anderen Zahnrades nur in einer Umdrehungsrichtung freigegeben, in der entgegengesetzten jedoch festgelegt. Das hat zur Folge, daß bei Drehung des Griffes 1 in der einen Richtung die Welle 2 raumfest, bei Umdrehung in die entgegengesetzte Richtung jedoch durch Eingriff des zugehörigen Plättchens 9 in das zugehörige Zahnrad 8 mitgeführt wird.

Dieser Drehsinn kann dadurch umgekehrt werden, daß durch eine hier nicht eingezeichnete Vorrichtung, beispielsweise durch einen Schieber das andere gegenüberliegende Plättchen 9 in Eingriffsstellung gebracht und das bisherige also gegenüberliegende freigegeben wird. Entscheidend ist für die Erfindung, daß in einer Endstellung stets in eines der beiden Zahnräder 8a, b eines der Plättchen 9a, b eingreift, das gegenüberliegende jedoch freigegeben wird.

Der hier nicht gezeigte Schieber sowie die Plättchen 9 bilden in ihrer Gesamtheit in funktioneller Hinsicht gesehen eine Sperrklinke, von der jeweils eines der beiden Plättchen 9a, 9b, das zugehörige Zahnrad 8a, 8b freigibt, das ander Plättchen 9b, 9a jedoch in das zugehörige Zahnrad 8b, 8a eingreift und hierdurch den Drehsinn festlegt, bei welchem der Griff 1 die Welle 2 mitnimmt. Der Vorteil der erfindungsgemäßen Anordnung besteht darin, daß die Hand den Griff 1 umschließt und in eine oszillatorische Hin- und Herbewegung versetzen kann, hierdurch das Werkzeug in einer Richtung bewegt, ohne daß ein Umgreifen erforderlich wird. Durch ein Umschalten der Sperrlinke kann die Welle 2 bei gleicher Handhabung in entgegengesetzter Richtung bewegt werden. Die Vorrichtung erlaubt damit ohne Umgreifen zu müssen ein rasches und unter Beibehaltung einer präzisen Ausrichtung exaktes Arbeiten. Dies ist insbesondere für operative Zwecke wie dem der Osteosynthese, von besonderer Wichtigkeit.

Etwa im vorderen Drittel des Griffes 1 befindet sich eine Schnellwechselvorrichtung 11, die in axiale Richtung zur Welle 2 verschiebbar ist und sich hierbei an einer Feder 12 abstützt. Aufgabe der Schnellwechselvorrichtung besteht in der Herstellung einer Verbindung zu dem jeweils am Griff 1 befestigten Werkzeug, worunter Schraubenzieherklingen, Bohrer, Gewindeschneider und andere Werkzeuge zu verstehen sind. Sie dient der Aufnahme des Werkzeuges und dadurch der Herstellung einer Verbindung zur koaxial im Griff 1 gelagerten Welle 2. Die Schnellwechselvorrichtung soll das rasche Auswechseln der einzelnen Werkzeuge ermöglichen und ist von üblichem Aufbau.

## Patentansprüche

1. Chirurgisches Handinstrument insbesondere zur Osteosynthese mit einem Griff, an dem ein Werkzeug, wie Schraubenzieherklinge, Bohrwerkzeug, Gewindeschneider befestigt ist, koaxial im Griff (1) eine Welle (2) drehbar gelagert ist, auf der zwei Zahnräder (8a, 8b) im Abstand befestigt sind, und im Griff (1) eine von außen betätigbare Sperrklinke angeordnet ist, die in der einen Endlage nur in den Zahnkranz des einen Zahnrades (8a, 8b) und in der anderen Endlage nur in den Zahnkranz des anderen Zahnrades (8b, 8a) eingreift, wobei die Sperrklinke die Rotation des Zahnrades (8a, 8b) in nur einer, bei beiden Zahnrädern (8a, 8b) jedoch in zueinander entgegengesetzter Drehrichtung zuläßt, und daß an der Stirnseite der Welle (2) das Werkzeug befestigt ist, **dadurch gekennzeichnet,** daß die Sperrklinke aus jeweils um eine sehnenartig im Griff (1) verlaufende Achse verschwenkbares und jeweils in ein Zahnrad (8a, 8b) eingreifendes Plättchen (9a, 9b) sowie einem von außen im Griff bewegbaren Schieber besteht, der in der Endposition jeweils ein Plättchen (9a, 9b) aus dem Zahnkranz herausschwenkt.

2. Instrument nach Anspruch 1, **dadurch gekennzeichnet,** daß der Schieber aus zwei Federelementen besteht, die sich in der Endposition gegen ein Plättchen (9a, 9b) pressen oder durch eine magnetische Kopplung zwischen Schieber und Plättchen (9a, 9b).

3. Instrument nach Anspruch 1 oder 2, **dadurch gekennzeichnet,** daß in Mittelstellung des Schiebers beide Plättchen (9a, 9b) eingreifen.

4. Instrument nach einem der Ansprüche 1 bis 3, **dadurch gekennzeichnet,** daß der Zahnkranz beider Zahnräder (8a, 8b) Sägezahnprofil besitzt und die Flanken gleichen Anstiegs auf entgegengesetzten Seiten liegen.

5. Instrument nach einem der Ansprüche 1 bis 4, **gekennzeichnet durch** unterschiedliche Schrittweite der beiden Zahnkränze.

6. Instrument nach einem der Ansprüche 1 bis 5, **dadurch gekennzeichnet,** daß anstelle des Zahnrades (8a, 8b) zwei koaxiale Zahnradwalzen mit gegeneinander azimutal versetzten Zahnkränzen angeordnet sind, denen jeweils eine Sperrklinke zugeordnet ist.

7. Instrument nach Anspruch 6, **dadurch gekennzeichnet,** daß eine der Zahnradwalzen azimutal federnd gelagert ist.

8. Instrument nach Anspruch 7, **dadurch gekennzeichnet,** daß die azimutale Bewegung maximal einen Zahnabstand beträgt.

9. Instrument nach einem der Ansprüche 1 bis 8, **gekennzeichnet durch** eine im Vorderteil des Griffs (1) befindliche Betätigung der Sperrklinke.

10. Instrument nach einem der Ansprüche 1 bis 9, **gekennzeichnet durch** einen auf der Welle (2) befestigten Drehmomentenbegrenzer.

11. Instrument nach Anspruch 10, **dadurch gekennzeichnet,** daß der Drehmomentenbegrenzer aus zwei aufeinander gepreßten Kupplungsscheiben (4, 5) besteht, von der eine mit der Welle (2) und die andere mit dem Griff (1) in Verbindung steht.

12. Instrument nach Anspruch 11, **dadurch gekennzeichnet,** daß die Kupplungsscheiben (4, 5) mit aufeinander zu weisendem Zahnprofil, insbesondere Sägezahnprofil, versehen sind und durch eine Feder (7) gegeneinander gepreßt werden und/oder federbelastete, in Nuten und/oder Zahnkränzen eingreifende Kugeln aufweisen.

13. Instrument nach Anspruch 12, **gekennzeichnet durch** einstellbare Vorspannung der Feder (7).

14. Instrument nach einem der Ansprüche 1 bis 13, **gekennzeichnet durch** eine auf der Welle (2) angebrachte schnellwechselvorrichtung (11).

## Claims

1. Hand-held surgical instrument especially for osteosynthesis having a handle, on which is fastened a tool, such as a screw driver blade, drilling tool, thread cutter, coaxially a shaft (2) is mounted so as to be rotatable in handle (1), upon which, at a distance to each other, are fastened two toothed wheels (8a, 8b), and a detent pawl that can be operated from outside is disposed in handle (1), which in one stop position engages only with the rim of one of the toothed wheels (8a, 8b) and in the other stop position only with the rim of the other toothed wheel (8b, 8a), whereby the detent pawl permits the rotation of the toothed wheel (8a, 8b) in one direction only, in the case of both toothed wheels (8a, 8b), however, in a direction of rotation that is opposed to each other, and that on the front end of the shaft (2) the tool is fastened, **wherein** said detent pawl consists of a small plate (9a, 9b), one of which can be swivelled about an axis extending in a chordal manner in said handle (1), the other engaging with a toothed wheel (8a, 8b), as well as a slide in the handle that can be moved from outside, which in the end position swings out one of the respective small plates (9a, 9b) from the rim of the toothed wheel.

2. Instrument according to claim 1, **wherein** said slide consists of two spring elements, which in the end position press themselves against a small plate (9a, 9b) or through a magnetic coupling between said slide and small plate (9a, 9b).

3. Instrument according to claim 1 or 2, **wherein** in the mid-position of said slide both small (9a, 9b) engage.

4. Instrument according to one of claims 1 to 3, **wherein** said rim of both toothed wheels (8a, 8b) has a saw-tooth profile and on opposing sides the tooth flanks have the same inclination.

5. Instrument according to one of claims 1 to 4, **wherein** both toothed wheel rims have different step widths.

6. Instrument according to one of claims 1 to 5, **wherein** instead of said toothed wheel (8a, 8b) two coaxial toothed wheel rolls are disposed so that the rims are staggered azimuthally, to each of which is assigned a detent pawl.

7. Instrument according to claim 6, **wherein** one of said toothed wheel rolls is spring-mounted azimuthally.

8. Instrument according to claim 7, **wherein** azimuthal movement amounts maximally to the distance represented by a tooth.

9. Instrument according to one of claims 1 to 8, **wherein** the means of operation of said detent pawl is disposed in the head of said handle (1).

10. Instrument according to one claims 1 to 9, **wherein** a torque limiter is fastened on said shaft (2).

11. Instrument according to claim 10, **wherein** said torque limiter consists of two clutch disks (4, 5) that are pressed together, one of which is connected to said shaft (2), the other to said handle (1).

12. Instrument according to claim 11, **wherein** said clutch disks (4, 5) are provided with a tooth profile pointing toward each other, especially a saw-tooth profile, and are pressed together by a spring (7) and/or comprise spring-weighed balls that engage in grooves and/or toothed wheel rims.

13. Instrument according to claim 12, **wherein** the means of prestressing of said spring (7) is adjustable.

14. Instrument according to one of claims 1 to 13, **wherein** a quick-change device (11) is disposed on said shaft (2).

## Revendications

1. Instrument chirurgical à usage manuel, en particulier pour ostéosynthèse, muni d'un manche auquel est fixé un outil, tel qu'une pointe de tournevis, un foret, un taraud, d'un arbre (2) supporté en rotation de façon coaxiale dans le manche (1) et sur lequel sont fixées deux roues dentées (8a,8b) à distance l'une de l'autre, d'un cliquet d'arrêt disposé dans le manche et actionnable de l'extérieur, et qui dans une position de fin de course n'engrène que dans la couronne dentée de l'une des roues dentées (8a,8b), et qui dans l'autre position de fin de course n'engrène que dans la couronne dentée de l'autre roue dentée (8b,8a), le cliquet d'arrêt n'autorisant la rotation de la roue dentée (8a,8b) que dans un seul sens de rotation, les sens de rotation autorisés étant pour les deux roues dentées (8a,8b) opposés l'un à l'autre, et l'outil étant fixé à la partie avant de l'arbre (2), **caractérisé en ce que** le cliquet d'arrêt est composé chaque fois d'une plaquette (9a,9b) pivotable autour d'un axe respectif s'étendant dans le manche (1) selon une corde de ce dernier et s'encliquetant dans une roue dentée (8a,8b) respective, ainsi que d'un poussoir sur le manche, manipulable de l'extérieur, et qui en fin de course désengage l'une des plaquettes (9a,9b) de la couronne dentée.

2. Instrument selon la revendication 1, **caractérisé en ce que** le poussoir se compose de deux éléments à ressorts, qui en position de fin de course exercent une pression sur une plaquette (9a,9b) ou par couplage magnétique entre poussoir et plaquette (9a,9b).

3. Instrument selon la revendication 1 ou 2, **caractérisé en ce que** dans la position intermédiaire du poussoir les deux plaquettes (9a,9b) s'encliquettent.

4. Instrument selon l'une des revendications 1 à 3, **caractérisé en ce que** la couronne dentée des deux roues dentées (8a,8b) a un profil en dents de scie, et les flancs de même montée sont situés dans des positions opposées.

5. Instrument selon l'une des revendications 1 à 4, **caractérisé par** des écartements de pas différents des deux couronnes dentées.

6. Instrument selon l'une des revendications 1 à 5, **caractérisé en ce que** à la place de la roue dentée (8a,8b) sont disposés deux rouleaux dentés coaxiaux à couronnes dentées décalées en azimut l'une par rapport à l'autre, à chacun desquels est associé un cliquet d'arrêt.

7. Instrument selon la revendication 6, **caractérisé en ce que** l'un des rouleaux dentés est supporté de façon élastique en azimut.

8. Instrument selon la revendication 7, **caractérisé en ce que** le mouvement azimutal est au maximum de la valeur d'un écartement de dent.

9. Instrument selon l'une des revendications 1 à 8, **caractérisé par** un actionnement du cliquet d'arrêt placé à l'avant du manche (1).

10. Instrument selon l'une des revendications 1 à 9, **caractérisé par** un réducteur de couple fixé à l'arbre (2).

11. Instrument selon la revendication 10, **caractérisé en ce que** le réducteur de couple se compose de deux plateaux d'accouplement (4,5) appliqués l'un contre l'autre, dont l'un est connecté à l'arbre (2) et l'autre au manche (1).

12. Instrument selon la revendication 11, **caractérisé en ce que** les plateaux d'accouplement (4,5) sont pourvus de profils dentés, en particulier de profils en dents de scie, tournes l'un vers l'autre, et sont appliqués l'un contre l'autre par un ressort (7) et/ou comportent des billes soumises à l'action de ressorts et reçues dans des rainures et/ou des couronnes dentées.

13. Instrument selon la revendication 12, **caractérisé par** une précontrainte réglable du ressort (7).

14. Instrument selon l'une des revendications 1 à 13, **caractérisé par** un dispositif d'interchangeabilité rapide (11) disposé sur l'arbre (2).
